# EUROPÄISCHE PATENTSCHRIFT

(11) **EP 0 815 117 B1**
(45) Veröffentlichungstag und Bekanntmachung des Hinweises auf die Patenterteilung: **02.01.2002**
(21) Anmeldenummer: 96905870.0
(22) Anmeldetag: 12.03.1996
(51) Int. Cl.: C07H 21/00, C12Q 1/68, C07H 19/04, C07D 405/04, C07D 487/04

(54) **C-NUKLEOSID-DERIVATE UND DEREN VERWENDUNG IN DER DETEKTION VON NUKLEINSÄUREN**
C-NUCLEOSIDE DERIVATIVES AND THEIR USE IN NUCLEIC ACID DETECTION
DERIVES DE C-NUCLEOSIDES ET LEUR UTILISATION DANS LA DETECTION D'ACIDES NUCLEIQUES

(30) Priorität: 14.03.1995 DE 19509038
(43) Veröffentlichungstag der Anmeldung: 07.01.1998
(73) Patentinhaber: Roche Diagnostics GmbH, 68298 Mannheim (DE)
(72) Erfinder: MÜHLEGGER, Klaus, D-82398 Polling (DE); VON DER ELTZ, Herbert, D-82362 Weilheim (DE); SEELA, Frank, D-49076 Osnabrück (DE); ROSEMEYER, Helmut, D-49076 Osnabrück (DE)
(86) Internationale Anmeldenummer: EP9601051
(87) Internationale Veröffentlichungsnummer: WO9628460

(56) Entgegenhaltungen:
- WO-A-93/16094
- WO-A-94/11534
- WO-A-95/05391
- US-A- 4 584 369
- J. CHEM. SOC. PERKIN TRANS. 1, 1984, Seiten 2421-3, XP002008298 H. DAVIES ET AL.: "Conversion of Formycin into the Fluorescent Isoguanosine Analogue 7-Amino-3-(beta-D-Ribofuranosyl)-1H-pyrazo lo(4,3-d)pyrimidin-5(4H)-one"
- J. ORG. CHEM., Bd. 53, 1988, Seiten 2777-82, XP002008299 C.K. CHU ET AL.: "Synthesis of C-Nucleoside Analogues"
- HELV. CHIM. ACTA, Bd. 77, 1994, Seiten 481-501, XP002008300 W. PFLEIDERER ET AL.: "44. Nucleotides Part XLII"
- NUCLEOSIDES, NUCLEOTIDES, Bd. 15, 1996, Seiten 793-807, XP002008301 B.A. OTTER ET AL.: "Conformational Properties of Purine-like C-Nucleosides"
- BIOCHIMIE, Bd. 77, 1995, Seiten 125-34, XP002008302 PF. AGRIS ET AL.: "Site-selected Introduction of modified purine and pyrimidine ribonucleosides into RNA by automated phosphoramidite chemistry "
- DATABASE PATENT ABSTRACTS OF JAPAN 1990 "Pseudouridine Derivative" XP002008303 & JP,A,02 196 787 (OTSUKA PHARMACEUT CO.) 3.August 1990
- DATABASE PATENT ABSTRACTS OF JAPAN 1989 "Pseudouridine Derivative" XP002008314 & JP,A,01 294 674 (OTSUKA PHARMACEUT. CO.) 28.November 1989
- NUCLEIC ACIDS RES., Bd. 20, 1992, Seiten 4831-7, XP002008312 K.J. LIVAK ET AL.: "Detection of single base differences using biotinylated nucleotides with very long linker arms" in der Anmeldung erwähnt
- BIOL. CHEM. HOPPE-SEYLER, Bd. 371, 1990, Seiten 953-65, XP002008313 E. HUBER ET AL.: "Non-radioactive Labeling and Detection of Nucleic Acids" in der Anmeldung erwähnt

## Beschreibung

Die Erfindung betrifft C-Nukleoside und deren Derivate sowie deren Verwendung zur Markierung, Detektion und Sequenzierung von Nukleinsäuren.

Nukleinsäuren haben als Träger bzw. Überträger der genetischen Information eine zentrale Bedeutung in der belebten Natur. Sie haben deshalb seit ihrer Entdeckung durch F. Miescher das breite Interesse der Naturwissenschaften erregt und zur Aufklärung ihrer Funktion, Struktur und Wirkungsweise geführt. Mit der zunehmenden Kenntnis dieser grundlegenden molekularbiologischen Mechanismen ist es in den letzten Jahren möglich geworden, die Neukombination von Genen zu betreiben. Diese Technologie eröffnet z.B.neue Möglichkeiten in der medizinischen Diagnose und Therapie und in der Pflanzenzüchtung.

Ein wesentliches Werkzeug zur Erklärung dieser Zusammenhänge und der Lösung der Probleme war und ist der Nachweis der Nukleinsäuren und zwar sowohl was ihren spezifischen Nachweis betrifft, als auch was ihre Sequenz, also ihre Primärstruktur angeht.

Die spezifische Nachweisbarkeit von Nukleinsäuren beruht auf der Eigenschaft dieser Moleküle, mit anderen Nukleinsäuren durch Ausbildung von Basenpaarungen über Wasserstoffbrücken in Wechselwirkung zu treten, zu "hybridisieren". In geeigneter Weise markierte, d. h. mit Indikatorgruppen versehene Nukleinsäuren ("Sonden") können so zum Nachweis komplementärer Nukleinsäuren ("target") eingesetzt werden.

Die Ermittlung der Primärstruktur ("Sequenz"), also der Abfolge der heterocyclischen Basen einer Nukleinsäure geschieht mittels den Techniken der "Sequenzierung". Diese Kenntnis der Sequenz ist wiederum die Grundvoraussetzung für einen gezielten und spezifischen Einsatz von Nukleinsäuren in molekularbiologischen Fragestellungen und Arbeitstechniken. Auch die Sequenzierung bedient sich letztlich des Prinzips der spezifischen Hybridisierung von Nukleinsäuren untereinander. Dabei werden wie oben erwähnt ebenfalls markierte Nukleinsäure-Fragmente verwendet.

Demzufolge ist die geeignete Markierung von Nukleinsäuren eine unverzichtbare Voraussetzung jeglicher Nachweismethode.

Schon frühzeitig wurde dafür vor allem die radioaktive Markierung mit geeigneten Isotopen, wie ³²P oder ³⁵S eingesetzt. Die Nachteile der Verwendung radioaktiver Reagentien liegen jedoch klar auf der Hand: entsprechende Arbeiten bedürfen spezieller räumlicher Einrichtungen und Genehmigungen, sowie einer kontrollierten und aufwendigen Entsorgung des radioaktiven Abfalls. Die Reagentien zur radioaktiven Markierung sind zudem kostspielig. Eine längere zeitliche Aufbewahrung derart markierter Proben ist wegen der kurzen Halbwertszeit obiger Nuklide nicht möglich.

Es hat daher in den letzten Jahren nicht an Versuchen gefehlt, diese gravierenden Nachteile zu umgehen, d. h. von einer radioaktiven Markierung wegzukommen. Dabei sollte die hohe Sensitivität dieser Markierungsart möglichst beibehalten werden.

Hier sind in der Tat bereits große Fortschritte erzielt worden [siehe z.B."Nonradioactive Labeling and Detection of Biomolecules" (Kessler, C., Hrsg.) Springer Verlag Berlin, Heidelberg 1992].

Eine unabdingbare Voraussetzung jeglicher Detektion einer Nukleinsäure ist die vorherige Markierung derselben, und zwar - wie oben ausgeführt - sollte dies möglichst in einer nichtradioaktiven Art und Weise erreicht werden. Während die radioaktive Markierung der Nukleinsäuren in der Regel durch enzymatisch katalysierten Einbau entsprechender radioaktiver Nukleosid-triphosphate erfolgt, muß eine nicht-radioaktive Markierung über den Einbau einer geeigneten Signal- oder Reportergruppe geschehen.

Als nicht-radioaktive Indikatormoleküle haben sich u. a. hauptsächlich Haptene (wie Biotin oder Digoxigenin), Enzyme (wie alkalische Phosphatase oder Peroxidase) oder Fluoreszenzfarbstoffe (wie Fluorescein oder Rhodamine) bewährt. Diese Signalgruppen können nach verschiedenen Methoden an oder in Nukleinsäuren gebracht werden.

Eine relativ einfache Verfahrensweise z.B.ist die Markierung am 5'-Ende eines mit einer endständigen Aminofunktion versehenen Oligonukleotides mittels aktivierter Indikator-moleküle der oben genannten Art. Sie erlaubt aber nur das Einführen eines oder weniger Indikatormoleküle in ein nur niedermolekulares Oligomer, während eine dichtere Markierung längerkettiger, hochmolekularer Nukleinsäuren mit dem Ziel einer hohen Sensitivität in der Regel über den Einbau von mit Reportergruppen versehenen Nukleosidtriphosphaten mittels Polymerasen im Sinne einer *de novo* -Synthese erfolgen muß.

Entsprechende Verfahren sind dem Fachmann als "nick translation" [Rigby, P. W. et al. (1977) J. Mol. Biol. **113,** 237] und "random primed labeling" [Feinberg, A. P. & Vogelstein, B. (1984) Anal. Biochem. **137,** 266] bekannt. Eine weitere Methode ist die sogenannte 3'-tailing-Reaktion mit Hilfe des Enzyms "Telminale Transferase".

Die bislang in diesen Verfahren eingesetzten Nukleosid-triphosphate sind nahezu ausschließlich entsprechend modifizierte Derivate der heterocyclischen Basen Adenin, Guanin, Cytosin und Thymin in der Desoxyribonukleotid-, bzw. Adenin, Guanin, Cytosin und Uracil in der Ribonukleotid-Reihe. Solche Derivate sind z.B.von Langer et al., Proc. Natl. Acad. Sci. USA **78**, 6635 (1981); Mühlegger et al., Biol. Chem. Hoppe-Seyler **371**, 953 (1990) und in EP 0 063 879 beschrieben. Hier werden die natürlicherweise in DNA und RNA vorkommenden Bausteine in markierter, d. h. mit Signalgruppen versehener Form eingesetzt. Die wesentlichen Nachteile der N-Nukleoside liegen in der Empfindlichkeit der N-glykosidischen Bindung gegenüber sauren pH-Bedingungen und der Abbaubarkeit durch Nukleasen.

Ferner sind einzelne C-Nukleoside (siehe z.B.Suhadolnik, R.J. in "Nucleoside Antibiotics", Wiley-Interscience, New York 1970) und deren Verwendung im therapeutischen (antiviralen oder cancerostatischen) Bereich seit längerem bekannt.

Außerdem sind fluoreszierende C-Nukleosid-Derivate und deren Einbau in DNA- bzw. RNA-Oligonukleotide beschrieben (WO 93/16094). Ebenfalls bekannt sind entsprechende mit einem Chromophor markierte Pseudouridine (Helv. Chim. Acta **77**, S. 481-501 (1994)). Die sogenannte Eigenfluoreszenz dieser C-Nukleoside ist jedoch bezüglich der Quantenausbeute um ein Vielfaches geringer als die spezieller Fluorophore wie z.B.Fluorescein oder entsprechende Rhodaminderivate. Ein weiterer Nachteil der selbstfluoreszenten C-Nukleoside liegt in ihren vergleichsweise niedrigen Anregungs- und Emissionswellenlängen. Dies hat zur Folge, daß Detektionssysteme, welche auf solchen Derivaten beruhen, nur eine geringe Nachweisempfindlichkeit besitzen, zum anderen machen sich spektral störende Einflüsse der Meßumgebung (wie biologisches Material, Autofluoreszenz von Gelmatrices etc.) sehr stark bemerkbar.

Die bekannten Nukleoside bzw. Nukleosid-Derivate weisen somit eine Reihe von Nachteilen auf, die sich insbesondere für den Nachweis von Nukleinsäuren negativ auswirken.

Der Erfindung liegt somit die Aufgabe zugrunde mit Signalgruppen modifizierte Nukleosid-Derivate für die Detektion von Nukleinsäuren zur Verfügung zu stellen, die die genannten Nachteile nicht aufweisen, d. h. insbesondere stabiler sowie zugleich enzymatisch prozessierbar und für den Nachweis von Nukleinsäuren bei einer praktikablen Wellenlänge geeignet sind.

Die Aufgabe wird durch Pyrimidinfuranoside der allgemeinen Formel (I): 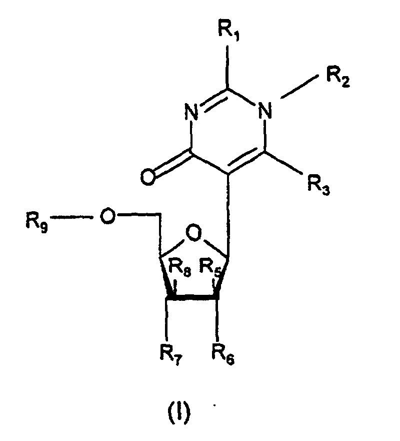 gelöst, in denen
- R₁, R₂, R₃,: die gleich oder verschieden sein können, Wasserstoff, Halogen, Hydroxy, Thio- oder substituiertes Thio-, Amino- oder substituiertes Amino-, Carboxy-, Niederalkyl-, Niederalkenyl-, Niederalkinyl-, Aryl-, Niederalkyloxy-, Aryloxy-, Aralkyl-, Aralkyloxy-, oder eine Reportergruppe darstellen,
- R₅ und R₆: jeweils Wasserstoff, Hydroxy, Thio- oder substituiertes Thio-, Amino- oder substituiertes Amino-, Niederalkyloxy-, Niederalkenoxy-, Niederalkinoxy-, eine Schutzgruppe oder eine Reportergruppe darstellen,
- R₇: Wasserstoff, Hydroxy, Thio- oder substituiertes Thio-, Amino- oder substituiertes Amino-, eine Phosphoramidit-oder H-phosphonat-Funktion, einen in geeigneter Weise spaltbaren Ester- oder Amid-Rest oder eine Reportergruppe darstellt,
- R₆ und R₇: zusammen eine weitere Bindung zwischen C-2' und C-3' oder eine Acetalfunktion bilden,
- R₈: Wasserstoff oder eine Hydroxy-, Thio- oder substituierte Thio-, Amino- oder substituierte Amino-Gruppe darstellt,
- R₉: Wasserstoff, eine Mono-, Di- oder Triphosphatgruppe, oder die alpha-, beta- oder gamma-Thiophosphatanalogen dieser Phosphorsäureester oder eine Schutzgruppe darstellt,
sowie mögliche Tautomere und Salze derselben.

Als Reportergruppe kommen jegliche detektierbaren Gruppen, wie insbesondere Haptene, ein Fluorophor, eine Metall-delatidierende Gruppe, ein Lumiphor, ein Protein- oder ein Interkalator in Frage.

Bevorzugt sind solche Verbindungen der allgemeinen Formel (I), in denen die Acetalfunktion der Reste R⁶ und R⁷ mit einer Reportergruppe substituiert sind.

Des weiteren haben sich solche Verbindungen als besonders geeignet erwiesen, in denen die Reportergruppe über eine sogenannte Linkergruppe an den aglykonischen bzw. Furanosering gebunden ist. Entsprechende Linkergruppen sind dem Fachmann bekannt (siehe z.B.Mühlegger, K. et al. (1990) Biol. Chem. Hoppe-Seyler 371, 953-965 oder Livak, K. J. et al. (1992) Nucl. Acids Res. 20, 4831-4837).

Besonders bevorzugte Verbindungen der allgemeinen Formel (I) sind solche, in denen R₁ Hydroxy eine Thio- oder Aminogrupe, die gegebenenfalls substituiert sein können oder eine Reportergruppe, R₂ und R₃ Wasserstoff, Niederalkyl oder eine Reportergruppe, R₅ Wasserstoff, R₆ Wasserstoff oder Hydroxy, R₇ Wasserstoff, Hydroxy, Thio eine gegebenenfalls sustituierte Aminogruppe, ein Phosphoramidit oder eine Reportergruppe, R₆ und R₇ zusammen eine Acetalfunktion, R₈ Wasserstoff und R₉ eine Triphosphat-Funktion darstellen.

Die Synthese der neuen, modifizierten C-Nukleoside erfolgt zweckmäßigerweise, indem man von den natürlich vorkommenden Vorstufen ausgeht. So läßt sich beispielsweise Formycin A als Adenosin-Analogon zu Formycin B (einem Inosin-Analogon) desaminieren. Dieses wiederum kann halogeniert und weiter Nukleophil substituiert werden, wodurch eine Reihe interessanter neuer Verbindungen, wie die der erfindungsgemäßen Art, hergestellt werden können.

Die Synthese der wichtigen 2'-Desoxy-Nukleoside erfolgt durch Deoxigenierung der oben erwähnten natürlich vorkommenden Ribonukleoside wie z.B.Formycin A. Hier wird heute vorwiegend die Deoxigenierungsreaktion nach Barton angewandt (Barton, D. H. R. & Motherwell, W. B. (1981) Pure Appl. Chem. **53,** 15).

Des weiteren kann die chemische Synthese der C-nukleoside, wie beispielsweise von K. A. Watanabe in "Chemistry of Nukleosides and Nukleotides" **3**, 421-535 (L. B. Townsend, Hrsg.), Plenum Press, New York and London, 1994 eingehend beschrieben, erfolgen.

Die Verwendung der erfindungsgemäßen Verbindungen zur Markierung von Nukleinsäuren mit diversen, definierten Signalgruppen und damit die Detektion und Sequenzierung von Nukleinsäuren hat sich als besonders vorteilhaft erwiesen.

Die erfindungsgemäßen C-Nukleoside der allgemeinen Formeln (I) besitzen insbesondere gegenüber den klassischen N-glykosidisch verknüpften Nukleosiden und Nukleotiden wie Adenosin, Guanosin, Cytidin, Thymidin, Uridin bzw. deren entsprechenden Phosphorsäureestern eine Reihe von Vorteilen.

Ein Vorteil ist die chemische Stabilität der C-glykosidischen Bindung, z.B.gegenüber sauren pH-Bedingungen. Ein weiterer wesentlicher Vorteil ist die Stabilität dieser Verbindungen gegenüber dem enzymatischen Abbau durch Endo- und Exonukleasen. Diese Enzyme sind in biologischem Material enthalten und können den Nukleinsäure-Nachweis empfindlich stören. Andererseits ist es bekannt, daß DNA- und RNA-Polymerasen kritisch bezüglich der Akzeptanz mehr oder weniger modifizierter Nukleosid-5'-triphosphate sind, d.h. in der *de novo* Synthese solche Nukleotide als Substrate zu erkennen und einzubauen. Insbesondere das Anknüpfen von Signalgruppen an die Nukleotide beeinflußt erfahrungsgemäß Einbau und Einbaurate.

Daß die erfindungsgemäßen Nukleoside und ihre Derivate in sehr effizienter Weise durch geeignete Polymerasen in Nukleinsäuren eingebaut werden, wie z.B.nach den oben geschilderten Methoden der "nick translation" oder des "random primed labelling", ist nicht ohne weiteres aus dem Stand der Technik zu entnehmen und somit für den Fachmann als überraschend anzusehen.

Der genannten Verfahren bedient man sich ganz allgemein in der Nukleinsäure-Detektion, zB.beim quantitativen Nachweis nach Blotting-Techniken auf Membranen oder auch in Mikrotiterplatten.

Bei der Sequenzierung, d. h. dem Nachweis der Sequenz einer Nukleinsäure, wird an der zu sequenzierenden Nukleinsäure unter Zuhilfenahme eines kurzen (Start)Oligonukleotides ("primer") und einer Polymerase ein komplementärer Gegenstrang neusynthetisiert.

In der *in situ* -Hybridisierung zur Detektion bestimmter Gene oder Genomabschnitte läuft in der Zelle im Prinzip das gleiche ab, nämlich der spezifische Einbau von markierten Nukleotiden.

Die oben erwähnten Primer, d. h. kurzkettige Oligonukleotide, sollen, um eine optimale Funktion zu gewährleisten, sowohl stabile Basenpaarungen mit dem Matrizenstrang eingehen, als auch durch endogene Nukleasen nicht angegriffen werden.

Dies wird von Oligonukleotiden, welche anstatt der klassischen N-Nukleoside die erfindungsgemäßen C-Nukleoside als Bausteine enthalten, erfüllt.

Gleiches gilt für längerbettige Polynukleotide und Nukleinsäuren, die solche C-Nukleosidbausteine beeinhalten. Auch diese sind Gegenstand der vorliegenden Erfindung.

Entsprechende Oligonukleotide, sowie ihre präparativen Vorstufen der Nukleosidphosphoramidite und Nukleosid-H-phosphonate sind daher ebenfalls Gegenstand der Erfindung.

Oligonukleotide werden heute in der Regel nach bekannten Methoden in automatisch arbeitenden DNA/RNA-Synthesegeräten hergestellt.

Solche Syntheseverfahren basieren im wesentlichen auf der schrittweisen Umsetzung der o. a. Phosphoramidite oder H-Phosphonate und damit der fortlaufenden Verknüpfung dieser monomeren Bausteine zu Oligomeren (siehe z.B. T. Brown & D. J. S. Brown in "Oligonukleotides and Analogues-A Practical Approach" (1991) (Eckstein, F., Hrsg.), IRL Press at Oxford University Press, Oxford, New York, Tokyo).

Die folgenden Beispiele erläutern die Erfindung näher:

### Beispiel 1:

### 5-(2'-desoxy-β-D-erythro-pentofuranosyl) pyrimidin-2,4-dion-5'-triphosphat

650 mg (2,85 mmol) 5-(2'-Desoxy-β-D-erythro-pentofuranosyl)-pyrimidin-2,4-dion, hergestellt nach J. Org. Chem. **1982**, *47*, 485 durch Deoxigenierung des kommerziell erhältlichea 5-(β-D-erythro-pentofuranosyl) pyrimidin-2,4-dion (Pseudouridin), werden nach der Methode von Ludwig [Acta Biochim. et Biophys. Acad. Sci. Hung. (1981), **16,** 131] in einem Eintopf-Verfahren in das 5'-Triphosphat überführt. Anionenaustausch-Chromatographie an QAE-Sephadex mit einem LiCl-Gradient (Wasser auf 0,5 M) und Fällung in Aceton/Ethanol (2:1) und Trocknung ergeben 850 mg (60%) der Titelverbindung.
³¹P-NMR (0,1 M EDTA/D₂O/Eth₃N): -6,8 (d, P-γ); -11,0 (d, P-α); -22,0 (t, P-β).

### Beispiel 2:

### N¹-[Ethoxypropionyl]-5-(2'-desoxy-β-D-erythro-pentofuranosyl) pyrimidin-2,4-dion-5'-triphosphat

100 mg (0,2 mmol) des 2'-Desoxy-pseudouridin-5'-triphosphates aus Beispiel 14 werden in 5 ml 1 M Triethylammoniumbicarbonatpuffer (pH 8,9) gelöst und mit 5 ml Ethanol versetzt. Es werden 3 ml (ca. 30 mmol) Acrylsäureethylester zugefügt und die Mischung 6,5 h bei Raumtemperatur gerührt. Danach ist lt. DC (i-Buttersäure/konz. Ammoniak/ Wasser = 66/1/33) kein Edukt mehr zu beobachten. Das Reaktionsgemisch wird i.V. eingedampft und je lx mit einigen ml Ethanol und Wasser coevaporiert. Das rohe Produkt wird ohne weitere Reinigung wie unter Beispiel 16 angegeben, weiterverarbeitet.

### Beispiel 3:

### N¹-[1,3-Diaminopropyl]-5-(2'-desoly-β-D-erythro-pentofuranosyl)pyrimidin-2,4-dion-5'-triphosphat}

Das Produkt aus Beispiel 15 wird in 5 ml Wasser gelöst und mit 5 ml Ethanol, sowie 2 ml 1,3-Diaminpropan versetzt. Man rührt über Nacht bei Raumtemperatur und engt danach an der Ölpumpe i.V. bis zum viskosen Rückstand ein. Man nimmt in wenigen ml Wasser auf und stellt den pH mit verd. Essigsäure auf 6. Chromatographie/Entsalzung an RP 18 mit einem Triemyl-ammomumacetat/Acetonitril-Gradient lieferte 1000 A₂₆₀-Einheiten (ca. 0,1 mmol) des rohen Gemisches der Verbindung, aus dem nach Ionenaustausch-Chromatographie an QAE-Sephadex mit LiCl 140 A₂₆₀-Einheiten (ca. 20 µmol) der gewünschten Substanz isoliert wurden.
DC (i-Buttersäure/konz. Ammoniak/Wasser = 66/1/33) : R_{f}ca. 0,3; mit Ninhydrin positive Reaktion.

### Beispiel 4:

### N¹-Digoxigenin-3-O-methylcarbonyl-ε-aminocaproyl-1,3-diaminopropyl-propionyl]-5-(2'-desoxy-β-D-erythro-pentofuranosyl)pyrimidin-2,4-dion-5'-triphosphat

4,5 mg (5 µmol) des Diaminopropyl-Derivates aus Beispiel 16 werden in 1 ml 0,1 M Naborat-Puffer, pH 8,5 gelöst und dazu 5 mg (7,5 µmol) Digoxigenin-3-O-methylcarbonyl-ε-amino-capronsäure-N-hydroxysuccinimidester, gelöst in 1 ml aminfreiem Dimethylformamid gegeben. Man läßt die klare Lösung ca. 3 h bie Raumtemperatur stehen. Die Reaktionslösung wird danach eingedampft, der Rückstand in 1 ml Wasser aufgenommen und mittels RP 18-Chromatographie gereinigt (Säule: Inertsil, 250 x 8 mm, Triethylammoniumacetat Acetonitril). Nach Abziehen der flüchtigen Komponenten im Vakuum und Lyophilisation erhält man 7 mg (90%) der Titelverbindung.

### Beispiel 5:

### Tetramethylrhodamin-5(6)-carboxamido-{N¹-[8-N-(3,6-dioxa)octyl-1-amidomethyl]-5-(2'-desoxy-β-D-erythro-pentofuranosyl)pyrimidin-2,4-dion-5'-triphosphat}

Die Titelverbindung wurde durch Umsetzung von 18 mg (30 µmol) des Triphoshates aus Beispiel 16 und 17 mg (32 µmol) Tetramethylrhodamin-5(6)-carbonsäure-N-hydroxy-succinimidester in 0,1 M Na-boratpuffer, pH 8,5/DMF analog Beispiel 17 hergestellt und aufgereinigt. Es wurden 10,5 mg des TMR-markierten Nucleotids erhalten.
Spektrale Daten (0,1 M Na-boratpuffer, pH 8,5): Excitationₘₐₓ [nm]: 551;
Emissionₘₐₓ: [nm]: 575

### Beispiel 6:

### Nichtradioaktive DNA-Markierung und -Nachweis durch Einbau von

### N¹-Digoxigenin-3-O-methylcarbonyl-ε-aminocaproyl-1,3-diaminopropyl-propionyl]-5-(2'-desoxy-β-D-erythro-pentofuranosyl) pyrimidin-2,4-dion-5'-triphosphat]

Die DNA-Markierung und der DNA-Nachweis wurden mit dem kommerziell erhältlichen Kit der Firma Boehringer Mannheim (Best. Nr. 1093 657) durchgeführt. Die Arbeitsvorschrift gibt alle wesentlichen Arbeitsschritte wieder.

Zur Markierungsreaktion wurde das dNTP-Gemisch des Kits gegen ein solches mit N¹-[Digoxigenin-3-O-methylcarbonyl-ε-aminocaproyl-1,3-diaminopropyl-propionyl]-5-(2'-desoxy-β-D-erythro-pentofuranosyl) pyrimidin-2,4-dion-5'-triphosphat aus Beispiel 17 (statt DIG-dUTP) ausgetauscht.

Die immunologische Nachweisreaktion ergab, daß der Einbau der erfindungsgemäßen Verbindung nach Beispiel 17 eine Nachweissensitivität der markierten DNA analog der Verwendung von DIG-dUTP zeigt.

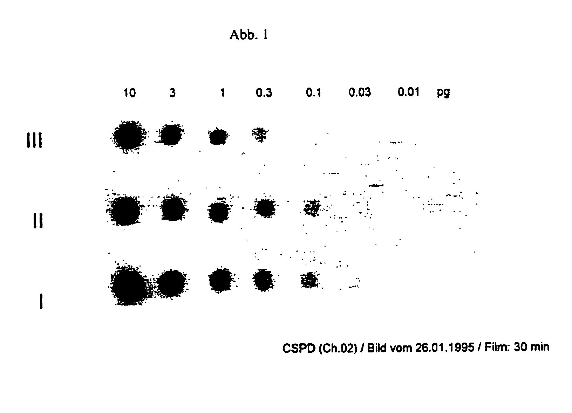

Das Ergebnis, den Nachweis und die erreichte Sensitivität des Systems demonstierend, ist Abbildung 1 zu entnehmen.

## Patentansprüche

1. Pyrimidin-furanoside der allgemeinen Formel (I) 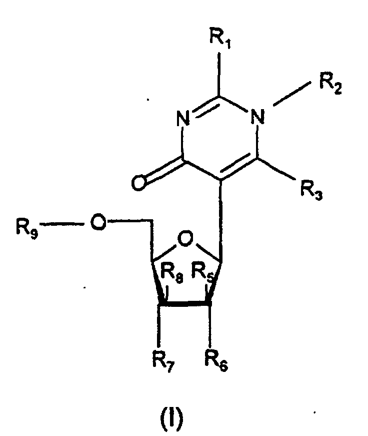 in denen
R₁, R₂, R₃, die gleich oder verschieden sein können, Wasserstoff, Halogen, Hydroxy, Thio- oder substituiertes Thio-, Amino- oder substituiertes Amino-, Carboxy-, Niederalkyl-, Niederalkenyl-, Niederalkinyl-, Aryl-, Niederalkyloxy-, Aryloxy-, Aralkyl-, Aralkyloxy-, oder eine Reportergruppe darstellen,
R₅ und R₆ jeweils Wasserstoff, Hydroxy, Thio- oder substituiertes Thio-, Amino- oder substituiertes Amino-, Niederalkyloxy-, Niederalkenoxy-, Niederalkinoxy-, eine Schutzgruppe oder eine Reportergruppe darstellen,
R₇ Wasserstoff, Hydroxy, Thio- oder substituiertes Thio-, Amino- oder substituiertes Amino-, eine Phosphoramidit-oder H-phosphonat-Funktion, einen in geeigneter Weise spaltbaren Ester- oder Amid-Rest oder eine Reportergruppe darstellt,
R₆ und R₇ zusammen eine weitere Bindung zwischen C-2' und C-3' oder eine Acetalfunktion bilden,
R₈ Wasserstoff oder eine Hydroxy-, Thio- oder substituierte Thio-, Amino- oder substituierte Amino-Gruppe darstellt,
R₉ Wasserstoff, eine Mono-, Di- oder Triphosphatgruppe, oder die alpha-, beta- oder gamma- Thiophosphatanalogen dieser Phosphorsäureester oder eine Schutzgruppe darstellt,
sowie mögliche Tautomere und Salze derselben, wobei wenigstens einer der Reste R₁ bis R₇ eine Reportergruppe darstellt, die an den Pyrimidin- oder Furanose-Ring über eine Linkergruppe gebunden ist.

2. Verbindungen nach Anspruch 1, in denen die Acetalfunktion mit einer Reportergruppe substituiert ist.

3. Verbindungen nach Anspruch 1 oder 2, in denen die Reportergruppe ein Hapten, Fluorophor, Metall-Chelat, Luminophor, Protein oder Interkalator bedeuten.

4. Verwendung der Verbindungen nach einem der Ansprüche 1 bis 3 als Substrate für DNA- und RNA-Polymerasen.

5. Verwendung der Verbindungen nach den Ansprüchen 1 bis 3 zur Markierung von Nukleinsäuren.

6. Verwendung der Verbindungen nach den Ansprüchen 1 bis 3 zum Nachweis von Nukleinsäuren.

7. Verwendung der Verbindungen nach einem der Ansprüche 1 bis 3 in der DNA-Sequenzierung.

8. Verwendung der Verbindungen nach einem der Ansprüche 1 bis 3 in der *in situ* Hybridisierung.

9. Verwendung der Verbindungen nach Anspruch 1, in denen R₇ ein Phosphoramidit oder H-Phosphonat darstellt, zur chemischen Synthese von Oligonukleotiden.

10. Oligonukleotide, die eine oder mehrere der in den Ansprüchen 1 bis 3 genannten Verbindungen enthalten.

11. Nukleinsäuren, die eine oder mehrere der in den Anspnichen 1 bis 3 genannten Verbindungen enthalten.

## Claims

1. Pyrimidine-furanosides of the general formula (I) 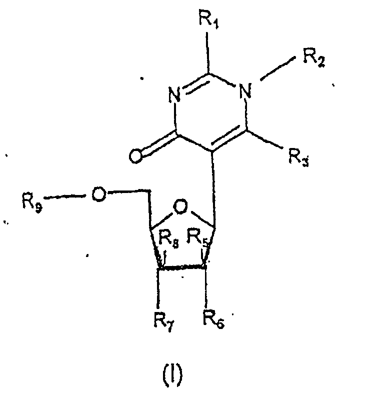 in which
R₁, R₂, R₃ can be same or different and represent hydrogen, halogen, hydroxy, thio or substituted thio, amino or substituted amino, carboxy, lower alkyl, lower alkenyl, lower alkinyl, aryl, lower alkyloxy, aryloxy, aralkyl, aralkyloxy or a reporter group,
R₅ and R₆ x each represent hydrogen, hydroxy, thio or substituted thio, amino or substituted amino, lower alkyloxy, lower alkenoxy, lower alkinoxy, a protecting group or a reporter group,
R₇ represents hydrogen, hydroxy, thio or substituted thio, amino or substituted amino, a phosphoramidite or H-phosphonate group, an ester or amide residue that can be cleaved in a suitable manner or a reporter group,
R₆ and R₇ together form a further bond between C-2' and C-3' or an acetal group,
R₈ represents hydrogen or a hydroxy, thio or substituted thio, amino or substituted amino group,
R₉ represents hydrogen, a mono-, di- or triphosphate group or the alpha, beta or gamma thiophosphate analogues of these phosphoric acid esters or a protecting group,
as well as possible tautomers and salts thereof, wherein at least one of the residues R₁ to R₇ represents a reporter group which is bound to the pyrimidine or furanose ring by means of a linker group.

2. Compounds as claimed in claim 1 in which the acetal function is substituted with a reporter group.

3. Compounds as claimed in claim 1 or 2 in which the reporter group denotes a hapten, fluorophore, metal chelate, luminophore, protein or intercalator.

4. Use of the compounds as claimed in one of the claims 1 to 3 as substrates for DNA and RNA polymerases.

5. Use of the compounds as claimed in claims 1 to 3 for labelling nucleic acids.

6. Use of the compounds as claimed in claims 1 to 3 for detecting nucleic acids.

7. Use of the compounds as claimed in one of the claims 1 to 3 in DNA sequencing.

8. Use of the compounds as claimed in one of the claims 1 to 3 in *in situ* hybridization.

9. Use of the compounds as claimed in claim 1, in which R₇ is a phosphoramidite or a H-phosphonate for the chemical synthesis of oligonucleotides.

10. Oligonucleotides which contain one or several of the compounds mentioned in claims 1 to 3.

11. Nucleic acids which contain one or several of the compounds mentioned in claims 1 to 3.

## Revendications

1. Pyrimidine-furanosides répondant à la formule générale (I) 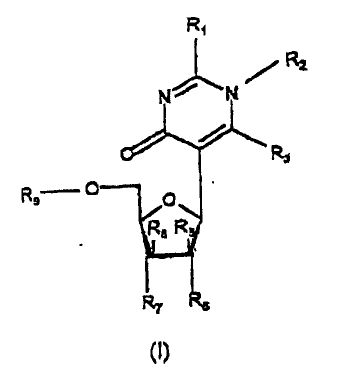 dans lesquels
R₁, R₂, R₃, qui peuvent être identiques ou différents, représentent un atome d'hydrogène, un atome d'halogène, un groupe hydroxyle, un groupe thio ou un groupe thio substitué, un groupe amino ou un groupe amino substitué, un groupe carboxyle, un groupe alkyle inférieur, un groupe alcényle inférieur, un groupe alcynyle inférieur, un groupe aryle, un groupe alkyle inférieur-oxy, un groupe aryloxy, un groupe aralkyle, un groupe aralkyloxy ou un groupe reporter,
R₅ et R₆ représentent respectivement un atome d'hydrogène, un groupe hydroxyle, un groupe thio ou un groupe thio substitué, un groupe amino ou un groupe amino substitué, un groupe alkyle inférieur-oxy, un groupe alcényle inférieur-oxy, un groupe alcynyle inférieur-oxy, un groupe de protection ou un groupe reporter,
R₇ représente un atome d'hydrogène, un groupe hydroxyle, un groupe thio ou un groupe thio substitué, un groupe amino ou un groupe amino substitué, une fonction phosphoramidite ou H-phosphonate, un radical ester ou amide qui peut être clivé de manière appropriée ou encore un groupe reporter,
R₆ et R₇ forment ensemble une liaison supplémentaire entre C-2' et C-3' ou encore une fonction acétal,
R₈ représente un atome d'hydrogène ou un groupe hydroxyle, un groupe thio ou un groupe thio substitué, un groupe amino ou un groupe amino substitué,
R₉ représente un atome d'hydrogène, un groupe monophosphate, diphosphate ou triphosphate, ou encore les analogues thiophosphates alpha, bêta ou gamma de ces esters phosphoriques ou encore un groupe de protection,
ainsi que les sels et les tautomères possibles des premiers cités, au moins un des radicaux R₁ à R₇ représentant un groupe reporter qui est lié au noyau pyrimidine ou au noyau furanose via un groupe de liaison.

2. Composés selon la revendication 1, dans lesquels la fonction acétal est substituée avec un groupe reporter.

3. Composés selon la revendication 1 ou 2, dans lesquels les groupes reporters représentent un haptène, un fluorophore, un chélate métallique, un luminophore, une protéine ou une substance intercalaire.

4. Utilisation des composés selon l'une quelconque des revendications 1 à 3, à titre de substrats pour des ADN- et ARN-polymérases.

5. Utilisation des composés selon l'une quelconque des revendications 1 à 3, pour le marquage d'acides nucléiques.

6. Utilisation des composés selon l'une quelconque des revendications 1 à 3, pour la détection d'acides nucléiques.

7. Utilisation des composés selon l'une quelconque des revendications 1 à 3, dans le séquençage d'ADN.

8. Utilisation des composés selon l'une quelconque des revendications 1 à 3, dans l'hybridation *in situ.*

9. Utilisation des composés selon la revendication 1, dans laquelle R₇ représente un phosphoramidite ou un H-phosphonate pour la synthèse *chimique* d'oligonucléotides.

10. Oligonucléotides qui contiennent un ou plusieurs des composés indiqués dans les revendications 1 à 3.

11. Acides nucléiques qui contiennent un ou plusieurs des composés indiqués dans les revendications 1 à 3.
